# EUROPÄISCHE PATENTSCHRIFT

(11) **EP 1 457 552 B1**
(45) Veröffentlichungstag und Bekanntmachung des Hinweises auf die Patenterteilung: **25.03.2009**
(21) Anmeldenummer: 04005863.8
(22) Anmeldetag: 12.03.2004
(51) Int. Cl.: C12N 5/00, D04H 11/00

(54) **Trägermaterial für die Gewebe- und Zellkultur, zur Herstellung von Implantaten oder Implantatmaterialien, und ein mit dem Trägermaterial hergestelltes Implantat**
Carrier for tissue- and cell-culture for the preparation of implants and implant containing the carrier
Support pour culture de cellules et tissus pour la préparation d'un implant et implant contenant cet support

(30) Priorität: 13.03.2003 DE 10312144
(43) Veröffentlichungstag der Anmeldung: 15.09.2004
(73) Patentinhaber: Technische Universität Dresden, 01062 Dresden (DE)
(72) Erfinder: Offermann, Peter, Prof. Dr., 01465 Liegau-Augustusbad (DE); Worch, Hartmut, Prof. Dr., 01187 Dresden (DE); Pompe, Wolfgang, Prof. Dr., 01737 Kurort Hartha (DE); Gelinsky, Michael, Dr., 01099 Dresden (DE); Schmack, Gerhilt, Dr., 01109 Dresden (DE); Hoffmann, Gerald, Dr., 01723 Wilsdruff (DE); Köckritz, Uwe, 01326 Dresden (DE)
(74) Vertreter: Kailuweit & Uhlemann Patentanwälte

(56) Entgegenhaltungen:
- EP-A- 0 692 227
- WO-A-87/00394
- DE-A- 2 533 682
- US-A- 4 016 303
- US-A- 5 326 357
- US-A- 6 123 727

## Beschreibung

Die Erfindung betrifft ein Trägermaterial für die Zell- und Gewebekultur (Tissue Engineering), zur Herstellung von Implantatmaterialien, wie insbesondere von Knochen- Knorpel- oder Hautersatz oder von extrakorporalem Organersatz oder zur anderweitigen Verwendung in der Medizin oder Biotechnologie. Die Erfindung betrifft auch ein mit dem Trägermaterial hergestelltes Implantat.

Es ist bereits bekannt, Zellen auf Trägermaterialien (Scaffolds) zu kultivieren und die so hergestellte Gewebekultur als Gewebeersatz zu verwenden. Als Trägermaterialien werden z. B. Gele, Faserstoffe, poröse Keramiken oder andere zwei- bzw. dreidimensionale Strukturen eingesetzt. Es ist bekannt, verschiedene Gewebe, wie z. B. Knorpel, die durch Verletzung, Abnutzung oder Krankheit defekt sind, mit Hilfe von gezüchteten Gewebekulturen zu therapieren. Hierzu werden körpereigene Zellen beispielsweise in dreidimensionale Trägermaterialien eingebracht und *in vitro* vermehrt und das so hergestellte Material in den Körper implantiert. Die für biokompatible Implantate eingesetzten Trägermaterialien haben wesentlichen Einfluss auf die Besiedelung, das Zellwachstum und die spätere Funktion des Implantates.

Faserstoffe bieten große Oberflächen für den Kontakt mit Zellen und bilden offenporige Strukturen, die für die Versorgung der Zellen mit Nährlösung notwendig sind. Häufig kommen heute Vliesstoffe zum Einsatz, die jedoch nachteilige Inhomogenitäten in der Struktur aufweisen. So werden größere Freiräume zwischen den Fasern nur sehr langsam von Zellen durchwachsen. In Bereichen, in denen die Fasern sehr dicht liegen ist die Zelldichte dagegen sehr gering. Eine Organisation der Faseranordnung in Vliesstoffen ist durch die starke Streuung in der Faserorientierung nur begrenzt möglich. Textile Flächengebilde aus Fäden sind zwar sehr gut strukturiert (s. o.), aber die Abstände zwischen den Fasern sind extrem unterschiedlich. Innerhalb der Fäden, die üblicherweise aus je 20 bis 200 Fasern bestehen, liegen die Fasern extrem dicht und zwischen den Fäden bestehen oft sehr große Abstände.

Trägermaterialien aus textilen Flächengebilden, insbesondere aus Vliesstoffen, Fasergeweben oder Maschenstoffen, weisen durch die überwiegend horizontale Anordnung der Fasern nur eine sehr geringe Druckstabilität auf. Aus DE 19959088 Al ist beispielsweise ein Melt-Blown-Verfahren zur Herstellung von dreidimensionalen luftverwirbelten Vliesen bekannt. Die Vliesstrukturen haben Porengrößen zwischen 20 µm und 500 µm und eine Porosität von < 95%.

In DE 197 216 61 A1 werden in einem dreidimensionalen Gitter angeordnete Stäbe zur Herstellung von Knochen- und Knorpelersatzstrukturen für den unmittelbaren Einsatz *in vivo* beschrieben. Diese Stäbe bestehen aus biopolymeren, thermoplastischen Werkstoffen und werden mittels selektiven Lasersinterns aus einer pulverförmigen Schicht oder in einem Three-Dimensional Printing (3-DP) Verfahren, ähnlich dem Funktionsprinzip eines Tintenstrahldruckers, schichtweise zu einem dreidimensionalen Gitter aufgebaut

US 4,016,303 offenbart eine Methode zur Beschichtung von Implantatoberflächen, die unmittelbar mit Blut in Kontakt stehen, mit Fasern. US 4,016,303 erwähnt das Problem, dass die auf die Oberfläche aufgebrachten Fasern sich ablösen und in die Blutbahn gelangen können. Die Fasern werden daher nach der erfolgten Beschichtung untereinander und mit der Substratoberfläche quervernetzt, um ein Loslösen der Fasern und eine Freisetzung in die Blutbahn zu vermeiden.

Aufgabe der vorliegenden Erfindung ist es, ein weiteres Trägermaterial für die Gewebe- und Zellkultur und zur Herstellung von Implantatmaterialien bzw. Implantaten und ein mit dem Trägermaterial hergestelltes Implantat anzugeben.

Erfindungsgemäß wird die Aufgabe gelöst durch ein Trägermaterial für die Gewebe- und Zellkultur nach Anspruch 1.

Das erfindungsgemäße Trägermaterial besteht ausschließlich aus biokompatiblen Materialien, d. h. Materialien, die ungiftig und mit biologischem Gewebe verträglich sind. Die Materialien werden bevorzugt so ausgewählt, dass sie den körpereigenen Materialien und Strukturen möglichst ähnlich sind, ohne immunogen oder allergen zu sein. Gleichzeitig werden die Materialien so ausgewählt, dass sie den Anforderungen des Flockprozesses gerecht werden.

Durch die elektrostatische Beflockung sind die Fasern nahezu senkrecht auf der Oberfläche des Basismaterials angeordnet. Vorteilhaft verhindert die durch die elektrostatische Beflockung erreichte hohe Faserauszugsfestigkeit ein Loslösen der Fasern von dem Basismaterial. Vorteilhaft ergibt das erfindungsgemäße Trägermaterial ein druckstabiles und elastisches Wachstumsgitter für die Zellbesiedlung *in vitro* bzw. das Einwachsen von Zellen *in vivo*. Die Fasern übernehmen dabei zusammen mit dem Basismaterial die Funktion der in natürlichem Gewebe vorkommenden extrazellulären Matrix. Die Faserdichte ist dazu mit über 100 Fasern/mm², vorzugsweise 150 bis 500 Fasern/mm², im Vergleich zu Flockstrukturen, die aus anderen Gebieten der Technik bekannt sind, recht hoch.

Die mittleren Abstände zwischen den biokompatiblen Kurzfasern im erfindungsgemäßen Trägermaterial (gemessen zwischen den Achsen der Fasern) liegen abhängig von den Zelltypen, für welche das Trägermaterial eingesetzt werden soll, zwischen 40 µm und 250 µm. Vorzugsweise haben die Fasern auf dem Basismaterial einen mittleren Abstand von 40 µm bis 130 µm. Die so gebildeten Zwischenräume zwischen den Fasern sind optimal für die Besiedlung des Trägermaterials mit Zellen.

Die hohe Druckstabilität (σ_{D} über 0,1 MPa linear elastisches Verhalten), und der hohe Druckmodul (E über 0,2 MPa) des Trägermaterials werden durch die nahezu senkrechte Ausrichtung der Fasern und die hohe Beflockungsdichte erzielt. Die erreichbare Beflockungsdichte ist umso höher, je kürzer die zur Beflockung eingesetzten Fasern sind. Bei 1 mm langen Fasern beträgt sie vorzugsweise 150 bis 250 Fasern/mm², bei 0,5 mm langen Fasern beträgt sie vorzugsweise 300 bis 400 Fasern/mm².
Je dichter die Beflockung und je kürzer die Fasern sind, umso weniger können die Fasern unter Druck zur Seite knicken, da sie von den benachbarten Fasern daran gehindert werden.

Das erfindungsgemäße biokompatible Trägermaterial wird nach dem aus der Textiltechnik bekannten Verfahren der elektrostatischen Beflockung hergestellt. Dabei werden biokompatible Fasern annähernd senkrecht auf ein biokompatibles Basismaterial aufgebracht. Die elektrostatische Beflockung wird vorzugsweise mit Gleichstrom durchgeführt. Um die erfindungsgemäß hohe Faserdichte zu erreichen, muss die elektrostatische Feldstärke variiert werden. Vorzugweise wird für die elektrostatische Beflockung das Basismaterial auf der zu beflockenden Seite mit einem Klebstoff beschichtet. Die Klebstoffbeschichtung ist entbehrlich, wenn das Basismaterial selbst eine klebrige Oberfläche aufweist.

In einer vorzugsweisen Ausführungsform ist das Basismaterial in einem frei definierbaren Muster beflockt. Durch definierte Bereiche mit und ohne Flockfasern wird die Zellbesiedlung gesteuert. Die Zellen siedeln sich bevorzugt auf den beflockten Bereichen, zwischen und auf den Fasern, an. Das Basismaterial ist einseitig beflockt oder auf mehreren Seiten. Eine partielle Beflockung bzw. eine Beflockung in frei definierbaren Mustern wird durch ein partielles Aufbringen des Klebstoffes, z. B. mittels Schablonen, vor der Beflockung erreicht.

In einer vorzugsweisen Ausführungsform der Erfindung bestehen Basismaterial, Klebstoff und/oder Fasern aus einem resorbierbaren Material. Unter resorbierbarem Material ist hier und im folgenden Text zu verstehen, dass das Material nach der Implantation im Körper langsam durch biologische oder biochemische Prozesse abgebaut wird bzw. sich langsam auflöst. Eine partielle Resorption des Materials kann auch während der Zellkultivierung *in vitro* einsetzen.

Als resorbierbares Material wird für das Basismaterial vorzugsweise eine Membran oder ein Tape aus Kollagen oder Kollagenderivaten, einschließlich mineralisiertem Kollagen, oder anderen Bestandteilen der extrazellulären Matrix, gewählt. In einer weiteren Ausführungsform der Erfindung ist das Basismaterial eine Membran oder ein Faserstoff, Vlies oder Gewebe aus resorbierbaren Biopolymeren (wie z. B. Chitosan, Hyaluronsäure, mineralisiertem und/oder nicht-mineralisiertem Kollagen- bzw. Kollagen-Hyaluronsäure-Kompositen), resorbierbaren aliphatischen Polyestern (z. B. Polylactid, Polyglycolid, Polyhydroxybutyrat, Polycaprolacton) bzw. deren Copolymere und Derivate aufgebaut.

In einer alternativen Ausführungsform besteht das Basismaterial aus einem nichtresorbierbaren biokompatiblen Material, wie z. B. einem biokompatiblen Metall (Titan, einer Titanlegierung, Stahl etc.), Kunststoff (Polymethylmethacrylat, Polystyrol, Polycarbonat etc.), Keramik (Calciumphosphat, Aluminiumoxid etc.) Glas oder Kohlenstoff (Kohlefaserverbundwerkstoff etc.).
Vorzugsweise hat das Basismaterial eine flache, wellige oder gewölbte Form, insbesondere in Form einer Membran (Tape). In einer alternativen Ausführungsform ist das Basismatrial ein dreidimensionaler Formkörper oder Hohlkörper. Vorzugsweise hat das Basismaterial eine profilierte Oberfläche. Das Basismaterial ist vorzugsweise so strukturiert, dass es Kräfte aufnimmt und das Trägermaterial bei Belastung zusammenhält.

In einer Ausführungsform ist das Basismaterial durch durch Stanzen bzw. mittels Wasserstrahl oder Laser gelocht. Vorzugsweise werden die Löcher in das Basismaterial nach der Beflockung eingebracht. Das Lochen nach der Beflockung hat den Vorteil, dass die Flockfasern am Lochrand nahezu senkrecht angeordnet sind.

Vorzugsweise wird als resorbierbarer Klebstoff Gelatine, ein Kollagen-Gel, Alginat-Gel Hyaluronsäure-Lösungen oder Chitosan-Lösungen verwendet. Als Klebstoff kommen aber auch andere biokompatible Materialien in Betracht, die eine gewisse elektrische Leitfähigkeit aufweisen und den Anforderungen an den elektrostatischen Flockprozess gerecht werden und somit zur Herstellung eines geeigneten Haftverbundes zwischen Basismaterial und Flockfaser geeignet sind. Vorzugsweise wird der Klebstoff nach der Beflockung quervernetzt. Die Quervernetzung wird durch chemische Crosslinker, wie z. B. EDC (N'-(3-Dimethylaminopropyl)-N-ethylcarbodiimid Hydrochlorid), oder andere bekannte Methoden, wie z. B durch UV- oder Gamma-Bestrahlung erreicht. Die Quervernetzung erfolgt insbesondere bei der Verwendung von Gelatine, einem Kollagengel oder Hyaluronsäure als Klebstoff, um ein schnelles Auflösen dieser Klebstoffe in wässriger Umgebung sowie ein Weichwerden während der Zellkultur bei 37 °C oder nach der Implantation zu vermeiden. Gleichzeitig wird durch den Crosslinker auch die Bindung zwischen Fasern, Klebstoff und Basismaterial verstärkt. Das Basismaterial und die Fasern haben vorzugsweise hydrophile Oberflächen, um eine gute Benetzung mit dem Klebstoff zu erreichen.

Die Fasermaterialien bestehen vorzugsweise aus resorbierbaren aliphatischen Polyestern, wie z. B. Polylactid (PLA), Polycaprolacton, Polyhydroxybutyrat und Polyglycolid, oder deren Derivate und Copolymere. Der Begriff Polyhydroxybutyrat (PHB) schließt dabei β- und γ-PHB, sowie deren Copolymere mit ein. Als Fasermaterialien werden in einer weiteren vorzugsweisen Ausführungsform auch nicht resorbierbare Faserstoffe eingesetzt, wie z. B. Polyacrylate, Polyamide, Polypropylen, Polytetrafluorethylen, Cellulose, Viskose oder andere Cellulosederivate, nicht bzw. langsam resorbierbare Polyester oder Kombinationen dieser Materialien, verwendet. Neben organischen Polymeren kommen auch anorganische Materialien wie Glas, Keramik, SiO₂ Kohlenstoff oder Metalle, oder Mischungen zum Einsatz.

Die Fasermaterialien müssen die für die elektrostatische Beflockung bekannten Eigenschaften aufweisen, wie z. B. einen ausreichenden elektrischen Oberflächenwiderstand von 10⁶ - 10⁸ Ω, der gegebenenfalls durch geeignete Präparationen bei der Fadenherstellung oder nachträglich erreicht wird. Der elektrische Widerstand der Flockfasern beeinflusst vor allem deren Ladungs- und Flugverhalten sowie die Gleichmäßigkeit der beflockten Flächen. Weiterhin hat das geometrische Verhältnis von Faserdurchmesser und Faserlänge einen wesentlichen Einfluss auf die Verarbeitungseigenschaften im Flockprozess. Es können Flockfasern mit einer Faserlänge zwischen 0,3 mm und 8 mm verarbeitet werden. Vorzugsweise weisen die einzelnen Fasern eine Länge zwischen 0,3 mm und 3 mm auf, insbesondere zwischen 0,5 mm und 1,5 mm. Der Durchmesser der einzelnen Fasern beträgt vorzugsweise zwischen 10 µm und 200 µm. Je kürzer die eingesetzten Fasern, desto höher ist die Druckstabilität des Trägermaterials.

In bevorzugten Ausführungsformen werden Fasern unterschiedlichen Materials, Durchmessers und/oder unterschiedlicher Länge kombiniert. Lange Flockfasern werden dabei durch kurze Fasern gestützt. Durch die Kombination unterschiedlich langer Fasern wird auch die erreichbare Faserdichte erhöht und eine unebene, strukturierte Oberfläche erreicht.

In einer weiteren vorzugsweisen Ausführungsform der Erfindung werden zur Reduzierung der Fasermasse und zur Steigerung der Druckelastizität Hohlfasern eingesetzt, welche offen- oder geschlossenporig sind.

In einer weiteren Ausführungsform ist das biokompatible Trägermaterial mindestens teilweise mineralisiert. Ein solches mineralisiertes Trägermaterial eignet sich insbesondere für den Einsatz als Knochenersatzmaterial. Bei einer teilweisen Mineralisierung sind vorzugsweise nur einzelne Komponenten, besonders vorzugsweise das Basismaterial mineralisiert. Bevorzugt wird das Trägermaterial oder Bestandteile desselben mit Hydroxylapatit (der Calciumphosphat-Phase des Knochens) mineralisiert. Die Herstellung eines teilweise mineralisierten Trägermaterials wird vorzugsweise dadurch erreicht, dass beim Aufbau des Trägermaterials mineralisierte Materialien, z. B. ein mineralisiertes Kollagen-Tape, als Basismaterial eingesetzt wird.

In einer weiteren Ausführungsform wird das fertig gebildete biokompatible Trägermaterial mineralisiert. Zu dieser Mineralisierung werden ein zur Mineralisierung von Kollagen bzw. Kollagentapes bekanntes Verfahren, wie z. B. die Abscheidung von Hydroxylapatit aus simulierter Körperflüssigkeit (SBF), (S.-H. Rhee, J. Tanaka: Hydroxyapatite coating on a collagen membrane by a biomimetic method. J. Am. Ceram. Soc. 1998, 81, 3029-3031) analog angewandt.

In einer besonderen Ausführungsform sind an das Basismaterial, den Klebstoff oder die Fasern Wirkstoffe gebunden. Unter Wirkstoffen sind hier z. B. pharmazeutische Stoffe, Peptide, Proteine, Antikörper, Wachstumsfaktoren, Cytokine, Chemokine, Oligonukleotide, Nukleinsäuren, wie cDNA, oder Nukleinsäurenderivate zu verstehen. Die Bindung dieser Stoffe an das jeweilige Material wird vorzugsweise durch kovalente Bindung an das Basismaterial oder Vermischen mit dem Basismaterial bzw. Klebstoff, vor der Beflockung erreicht. Alternativ erfolgt die Bindung dieser Stoffe an das Trägermaterial nach der Beflockung. Neben der kovalenten Anbindung können die Wirkstoffe auch adhäsiv oder mit anderen Methoden an das Trägermaterial oder dessen Komponenten, wie z. B. die Fasern, gebunden sein.

Ein weiterer Gegenstand der Erfindung ist ein mehrlagiges Trägermaterial (Mehrschicht-Scaffold), in dem mindestens zwei der oben beschriebenen biokompatiblen Trägermaterialien miteinander verbunden sind.

Durch den mehrlagigen Schichtaufbau werden durch die elektrostatische Beflockung definiert zellspezifische Porenabstände und definierte Lagenabstände erzeugt. Die Flockfasern wirken somit im mehrlagigen Aufbau als Abstandshalter.

In einer bevorzugten Ausführungsform des mehrlagigen Trägermaterials sind die einzelnen Trägermaterialien übereinandergestapelt. Dabei ergibt sich der Abstand zwischen den Basismaterialien der in Schichten angeordneten Trägermaterialien aus der Faserlänge zwischen den Basismaterialien. Vorzugsweise besteht die Verbindung zwischen den Trägermaterialien aus einer zusätzlichen Klebstoffschicht. Für den Klebstoff werden die oben genannten Materialien verwendet.

In einer weiteren Ausführungsform des mehrlagigen Trägermaterials werden die einzelnen Trägermaterialien mit Ihren Faserschichten ineinander gesteckt. Durch das Ineinanderstecken der Faserschichten bilden diese eine gemeinsame Faserschicht in der sich die Faserdichten addieren. Das Ineinanderstecken der Faserschichten hält vorteilhaft die beiden Trägermaterialien ohne eine zusätzliche Klebstoffschicht zusammen.

In einer besonders vorzugsweisen Ausführungsform enthält das mehrlagige biokompatible Trägermaterial Hohlräume, die untereinander verbunden oder voneinander isoliert sind.

Vorzugsweise wird durch diese ein Hohlraumsystem mit gestreckt, kanalförmig und/oder mäanderförmig verlaufenden, miteinander verbunden Hohlräumen gebildet.
Diese Hohlräume werden z. B. beim Zusammensetzen zu einem mehrlagigen Trägermaterial durch definierte Bereiche ohne Flockfasern auf den Basismaterialien und vorzugsweise durch den Einsatz definiert perforierter Basismaterialien gebildet. Diese durchgängigen und vorzugsweise dreidimensional miteinander verbundenen Hohlräume dienen unter anderem der Nährstoff- und Sauerstoffzufuhr zu den Zellen und ermöglichen ein schnelles Einsprossen von Blutkapillaren nach der Implantation.

Vorteilhaft kann das erfindungsgemäße biokompatible Trägermaterial als Implantatmaterial, zur Herstellung von Zell- und Gewebeimplantaten, sowie in der Zell- oder Gewebekultur und auch zur Herstellung eines extrakorporalen Organersatzes verwendet werden.

Bei der Verwendung als Implantatmaterial wird in einer vorzugsweisen Ausführungsform das Trägermaterial ohne vorherige Zellbesiedlung in den Körper implantiert. Nach der Implantation wird das Trägermaterial, in diesem Falle *in vivo,* mit Zellen, die aus dem umgebenden natürlichen Gewebe stammen, besiedelt. Diese Zellen wachsen dabei in die Flockfaserzwischenräume ein. Ist das Trägermaterial komplett aus resorbierbaren Materialien aufgebaut, wird es vorteilhaft innerhalb weniger Wochen bis weniger Jahre, bevorzugt innerhalb weniger Monate nach der Implantation resorbiert und durch körpereigenes Gewebe ersetzt.

In einer weiteren vorzugsweisen Ausführungsform der Erfindung wir das Trägermaterial *in vitro* mit Zellen besiedelt. Dazu werden Zellen für wenige Tage bis Wochen auf dem Trägermaterial kultiviert. Während dieser Zeit besiedeln Zellen das Trägermaterial bzw. wachsen in das Material ein. Dieses mit Zellen besiedelte Trägermaterial wird entweder als Gewebeersatz im Sinne des *Tissue Engineering* in den Körper implantiert oder es verbleibt für andere Aufgaben, wie den extrakorporalen Organersatz (z. B. eines temporären Leber-Ersatzes) außerhalb des Körpers.

In einer weiteren Variante wird das Basismaterial und/oder der Klebstoff des Trägermaterials nach oder während der Besiedlung mit Zellen entfernt, wenn die Zellen das Trägermaterial durch Sezernierung neugebildeter extrazellulärer Matrix soweit stabilisiert haben. Die Entfernung des Basismaterials bzw. Klebstoffes erfolgt mechanisch, chemisch, und/oder enzymatisch. Bestehen Basismaterial und/oder Klebstoff aus Kollagen, wird die Entfernung vorzugsweise durch Zugabe von Kollagenasen unterstützt.

Ein weiterer Bestandteil der Erfindung ist ein Implantat, welches das biokompatible Trägermaterial enthält. Dieses Implantat besteht aus einem- und/oder mehrlagigen Trägermaterial und vorzugsweise einer Hüllstruktur, welche das Trägermaterial umgibt.
Die Hüllstruktur ist aus biokompatiblen Werkstoffen aufgebaut. Vorzugsweise besteht sie aus textilen Fasern in Form eines Gewebes, Gestrickes, Gewirkes, Gestickes oder Vlieses. Die Fasern für die Hüllstruktur bestehen bevorzugt aus Biopolymeren, wie Chitosan, Polylactid, Polycaprolacton, Polyhydroxybutyrat und Polyglycolid, oder deren Derivaten und Copolymere. Alternativ besteht die Hüllstruktur aus einer perforierten Folie (oder einem perforierten Folienschlauch), die bevorzugt aus den oben genannten Biopolymeren aufgebaut ist. In einer weiteren Ausführungsform besteht die Hüllstruktur aus einem biopolymeren Tape aus Kollagen-Hyaluronsäure- oder Hydroxylapatit-Kollagen-Kompositen.

Das Implantat wird vorzugsweise vor der Implantation *in vitro* mit Zellen besiedelt. Dazu wird das Implantat, wie für das Trägermaterial oben beschrieben, mit Zellen besiedelt. Die Hüllstruktur bietet eine zusätzliche Stützfunktion und erleichtert die Handhabung des Implantats während der Zellkultur und während der Operation.

Bestandteil der Erfindung ist auch ein Verfahren zur Herstellung einer Gewebe- oder Zellkultur oder eines Implantatmaterials mit den folgenden Schritten:
a.) Herstellung eines erfindungsgemäßen Trägermaterials durch elektrostatisches Beflocken eines Basismaterials auf mindestens einer Seite mit Fasern,
b.)Inkubation des Trägermaterials mit Zellen zur Zellbesiedelung des Trägermaterials und zur Ausbildung einer extrazellulären Matrix, wobei nach der Ausbildung der extrazellulären Matrix das Basismaterials (1) gegebenenfalls wieder entfernt wird.

Zur Beflockung wird für den Fall, dass das Basismaterial nicht selbst eine klebrige Oberfläche aufweist, das Basismaterial zunächst mit Klebstoff beschichtet.

Durch die von den Zellen gebildete extrazelluläre Matrix werden die Fasern und Zellen zusammengehalten. Das zu Beginn bei der Beflockung als Trägerschicht für die Fasern notwendige Basismaterial und gegebenenfalls der Klebstoff wird daher vorzugsweise nach der Ausbildung der extrazellulären Matrix wieder entfernt.

Die extrazelluläre Matrix ist abhängig von dem zur Zellbesiedung eingesetzten Zelltyp aufgebaut. Sie ähnelt der in natürlichen Geweben vorkommenden extrazellulären Matrix. Bekannte Bestandteile der natürlichen extrazellulären Matrix sind beispielsweise Strukturproteine, wie Kollagene oder Elastin, Haftproteine, wie Laminin oder Fibronectin, sowie Polysaccharidkomponenten, wie Hyaluronsäure, bzw. Glycoproteine.

Die Entfernung des Basismaterials bzw. Klebstoffes erfolgt mechanisch, chemisch, und/oder enzymatisch. Bestehen Basismaterial und/oder Klebstoff aus Kollagen, wird die Entfernung vorzugsweise durch Zugabe von Kollagenasen unterstützt.

Ein weiterer Gegenstand der Erfindung ist ein Implantatmaterial, welches Zellen und eine extrazelluläre Matrix enthält. Dieses Implantatmaterial wird hergestellt durch die Schritte:
a.) Herstellung eines erfindungsgemäßen Trägermaterials durch elektrostatisches Beflocken eines Basismaterials (1) auf mindestens einer Seite mit Fasern (3),
b.) Inkubation des Trägermaterials mit Zellen zur Zellbesiedelung des Trägermaterials und zur Ausbildung einer extrazellulären Matrix,
c.) Entfernen des Basismaterials (1) nach der Ausbildung der extrazellulären Matrix.

Durch die elektrostatische Beflockung sind in diesem Implantatmaterial die Fasern vorteilhaft nahezu senkrecht und parallel zueinander angeordnet. Während der Inkubation siedeln sich die Zellen vorwiegend auf und zwischen den Fasern an und bauen eine extrazelluläre Matrix auf. Die Zellbesiedlung und der Aufbau der extrazellulären Matrix orientiert sich dabei an den Flockfasern. Vorteilhaft ergibt sich dadurch ein orientierter Gewebeaufbau, in dem Zellen und die extrazelluläre Matrix ähnlich einem natürlichen Gewebe zueinander orientiert sind.

Das so hergestellte Implantatmaterial eignet sich als Gewebeersatz, insbesondere als Knorpel- oder Hautersatz. Die zur Zellbesiedung eingesetzten Zellen werden dabei entsprechend dem zu ersetzenden Gewebe ausgewählt. So wird zur Herstellung eines Hautersatzmaterials das Trägermaterial z. B. mit Keranozyten und/oder Fibroblasten besiedelt. Für die Herstellung eines Knorpelersatzmaterials werden zur Zellbesiedlung Chondrozyten gewählt.

Die hohe Druckelastizität des Trägermaterials ermöglicht es vorteilhaft, eine mechanische Belastung ,des Zellverbands in vitro zu simulieren. In einer bevorzugten Ausführungsform erfolgt dazu während der Inkubation des Trägermaterials mit Zellen eine Applikation von Druck auf die Oberfläche des Trägermaterials. In einer alternativen Ausführungsform wird die Applikation von Druck durch eine Applikation von Ultraschall ergänzt oder ersetzt. Die mechanische Belastung *in vitro* kann dabei vorteilhaft so gewählt werden, dass sie der natürlichen Belastung von Zellen *in vivo,* wie z. B. der Chondrozyten im Gelenkknorpel, nahe kommt. Die Flockfasern simulieren dabei die natürliche extrazelluläre Matrix des Gewebes und nehmen einen Teil der Belastung auf.

Anhand nachfolgender Zeichnungen und Ausführungsbeispiele wird die Erfindung näher erläutert. Dabei zeigen
- Fig. 1.: Schnittdarstellung eines Trägermaterials
- Fig. 2.: Dreidimensionale Darstellung eines Trägermaterials
- Fig. 3.: Lichtmikroskopische Aufnahme eines horizontalen Querschnitts durch ein Trägermaterial
- Fig. 4.: Rasterelektronenmikroskopische Aufnahme eines Trägermaterials von der Seite
- Fig. 5.: Dreidimensionale Darstellung eines Trägermaterials mit beidseitig beflocktem Basismaterial
- Fig. 6.: Schnittdarstellung eines zweilagigen Trägermaterials
- Fig. 7.: Dreidimensionale Darstellung eines dreilagigen Trägermaterials
- Fig. 8.: Draufsicht auf ein partiell nicht beflocktes Trägermaterial
- Fig. 9.: Schnittebene durch ein mehrlagiges Trägermaterial mit gefäßartigen Hohlräumen
- Fig. 10.: Lichtmikroskopische Aufnahme einer mit Fibroblasten besiedelten PHB-Faser in einem Trägermaterial.

In den beigefügten Zeichnungen werden folgende Bezugszeichen verwendet:
- 1: Basismaterial
- 2: Klebstoff
- 3: Fasern
- 4: Bereiche ohne Fasern auf dem Basismaterial bzw. im Trägermaterial
- 5: Hohlräume

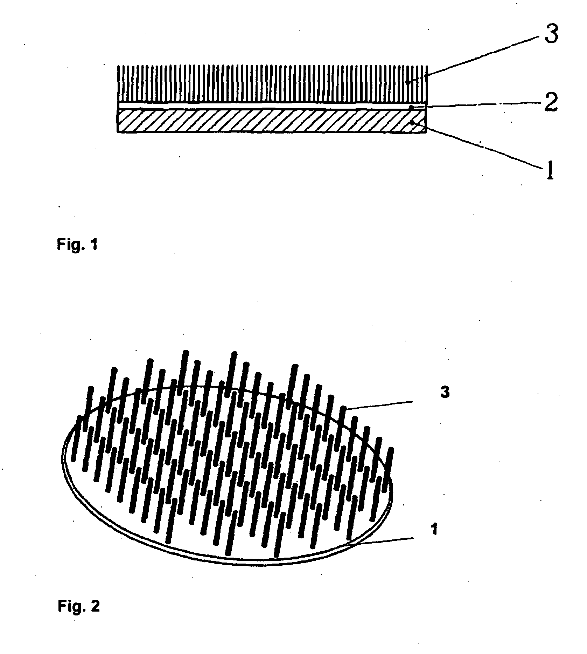

Das in **Fig. 1** dargestellte Trägermaterial besteht aus einem flachen Basismaterial 1, welches einseitig mit einer Klebstoffschicht **2** beschichtet und mit 1 mm langen Fasern **3** elektrostatisch beflockt ist.

Als biokompatibles, resorbierbares Basismaterial **1** wurde ein mineralisiertes Hydroxylapatit-Kollagen-Tape mit einem Durchmesser von 7 cm und einer mittleren Dicke von 0,2 mm ausgewählt. Das Hydroxylapatit-Kollagen-Tape wird aus mineralisiertem Kollagen (EP 0945146, EP 0945147, US 6384196, US 6384197, J.-H. Bradt, M. Mertig, A. Teresiak, W. Pompe: Biomimetic mineralization of collagen by combined fibril assembly and calcium phosphate formation. Chem. Mater. 1999, 11, 2694-2701) hergestellt, wie in (R. Burth, M. Gelinsky, W. Pompe: Collagen-hydroxyapatite tapes - a new implant material. Tech. Textile 1999, 8, 20-21) beschrieben.

Der Klebstoff **2** besteht aus Pharma-Gelatine, DGF Stoess AG, Eberbach, Deutschland, einem biokompatiblen, resorbierbaren Material.

Die Fasern **3** haben eine Länge von 1 mm und einen Durchmesser von 0,03 µm. Das Fasermaterial besteht aus Polyhydroxybutyrat, einem biokompatiblen und resorbierbaren Material mit einem E-Modul bis zu 7 GPa. Diese Kurzfasern erfüllen nach einer Oberflächenmodifizierung mittels alkalischer Hydrolyse (L. Rouxhet a.o. "Adsorption of albumin, collagen, and fibronectin on the surface of PHB/HV and of PCL films modified by an alkaline hydrolysis and of PET track-etched membranes", J. Biomater Sci. Polymer Edn. 9. (1998) 12, 1279-1304) die Anforderungen der Flocktechnologie an den elektrischen Oberflächenwiderstand von 10⁶-10⁸ Ω hinreichend.

### Zur Herstellung des Trägermaterials wird wie folgt verfahren:

Die Pharma-Gelatine **2** wird im Verhältnis 5 g : 100 ml mit destilliertem Wasser angesetzt, mittels Magnetrührer MR 3001 K und Temperaturregler EKT 3001, Heidolph Instruments GmbH & Co. KG, Schwabach, Deutschland, unter Rühren auf 60 °C erwärmt und nach vollständigem Auflösen der Gelatine auf 25 °C abgekühlt.

Fäden werden in einem Schmelzspinnprozess entsprechend der textilphysikalischen Vorgaben in einem online Spinnstreckprozesses erzeugt, entsprechend präpariert und anschließend zu 1mm langen Fasern **3** geschnitten.

Für die elektrostatische Beflockung wird eine Labor-Flockanlage der Fa, Maag Flockmaschinen GmbH, Iserlohn, Deutschland, verwendet. Die Anlagentechnik wurde für die Verarbeitung von 1 mm langen Kurzfasern **3**, durch die Auswahl einer geeigneten Siebelektrode mit 1,2 mm Lochdurchmesser und der Einstellung des Beflockungsabstandes auf 125 mm angepasst.

Die Pharma-Gelatine **2** wird gleichmäßig auf das Basismaterial **1** mittels Siebdruckschablone mit einer Klebstoffdicke **2** von 0,4 mm aufgebracht. Anschließend wird das mit Pharma-Gelatine **2** beschichtete Basismaterial **1** der Beflockungsanlage zugeführt. Die Flockspannung wird innerhalb von 20 Sekunden stufenlos von 20 kV auf 60 kV gesteigert, um hohe Flockdichten zu erreichen. Das Hochspannungsfeld wird noch weitere 10 Sekunden aufrechterhalten, um überschüssige Kurzfasern 3 durch die Feldwirkung von dem Trägermaterial zu lösen.

Anschließend werden die mit biokompatiblen und resorbierbaren Kurzfasern 3 so beflockten Hydroxylapatit-Kollagen Tapes gefriergetrocknet. Damit beim Trocknungsprozess auftretende Eigenspannungen nicht zum Verwerfen der Tapes führen, werden diese während des Trocknens mit porösen Druckplatten beschwert.
Durch das Gefriertrocknen schrumpft die Klebstoffdicke auf 0,02 mm.

Nach dem Gefriertrocknen wird die Klebschicht **2** der nun beflockten Hydroxylapatit-Kollagen-Tapes durch Einlegen für 4 Stunden in eine 1 %-igen Lösung von EDC N'-(3-Dimethylaminopropyl)-N-ethylcarbodiimid Hydrochlorid, Sigma-Aldrich Chemie GmbH, München, Deutschland, in 80 %-igem Ethanol chemisch quervernetzt. Bei der Quervernetzung werden kovalente Bindungen zwischen den Kollagenmolekülen der Gelatine-Klebschicht **2** und in geringerem Ausmaß auch zwischen den Kollagenmolekülen der Klebeschicht und dem Basismaterial 1 eingeführt, welche das Trägermaterial zusätzlich stabilisieren.

Das so hergestellte Trägermaterial zeichnet sich durch eine Flockfaserdichte von 290 Fasern/mm², einer Druckstabilität von σ_{D} = 0,13 MPa (linear elastisches Verhalten), einem Druckmodul von E = 0,25 MPa, einer Faserauszugsfestigkeit von σ_{F} = 0,41 MPa und Faserabstände zwischen 35 µm und 70 µm aus.

Vorteilhaft verhindert die hohe Faserauszugsfestigkeit ein Loslösen einzelner Fasern **3** vom Basismaterial **1** unter Zugbelastung.

Besonders vorteilhaft ist die hohe Druckstabilität und die Elastizität des Trägermaterials. Diese Werte erfüllen die mechanischen Anforderungen, die an Stützstrukturen in natürlichen Geweben und Organen, einschließlich Knorpel, gestellt werden.

Zur Besiedelung mit Zellen wird aus dem so hergestellten beflockten Hydroxylapatit-Kollagen-Tape **1** eine Probe mit einem Durchmesser von 13 mm ausgestanzt. Die Probe wird durch Bestrahlung mit Gamma-Strahlung sterilisiert und anschließend für 24 Stunden in 5 ml sterilem Zellkulturmedium (DMEM, Sigma GmbH, München, Deutschland) eingelegt. Das Medium wird alle 6 Stunden ausgetauscht.

Anschließend wird die Probe aus dem Medium herausgenommen und durch Auflegen auf ein steriles, saugfähiges Tuch etwas abgetrocknet. Die Probe wird nun mit der beflockten Seite nach oben in eine übliche 24er-Well-Zellkulturplatte überführt und eine Suspension von 1 × 10⁵ primären, humanen Chondrozyten in 1 ml Zellkulturmedium wird mittels einer Pipette aufgebracht. Die Zellkulturplatte wird nun für 24 h in einem üblichen Zellkultur-Brutschrank bei Standardbedingungen inkubiert. Während dieser Zeit erfolgt die Adhäsion der Chondrozyten an das Trägermaterial. Anschließend wird das so besiedelte Trägermaterial in frisches Medium überführt und bis zur weiteren Verwendung bei Mediumwechsel drei Mal wöchentlich unter Standardbedingungen weiter kultiviert.

Das Trägermaterial in **Fig. 2** ist mit dem in Fig. 1 identisch. Die Klebstoffschicht 2 wurde aus Gründen der Übersicht nicht dargestellt.

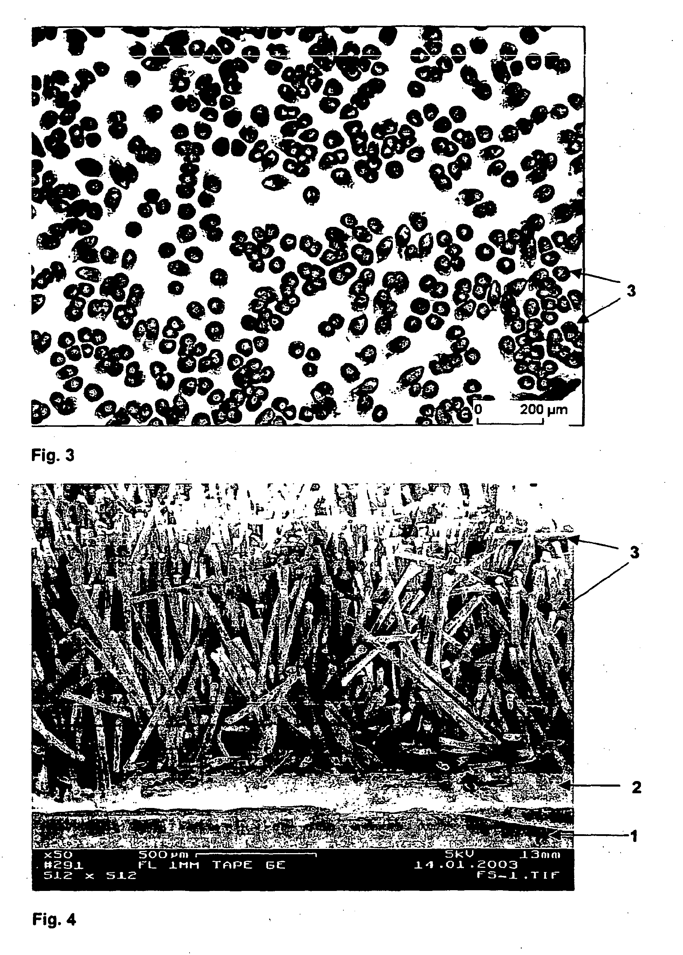

Für die mikroskopische Aufnahme (**Fig. 3**) wurde das Trägermaterial in Epoxidharz (SpeciFix-20, Fa. Struers) parallel zur Oberfläche eingebettet und in einem Schleif- und Poliervorgang für die optische Auswertung der Faserabstände vorbereitet. Die lichtmikroskopische Aufnahme wurde am Auf- und Durchlichtmikroskop AxioTech 100, Fa. Zeiss (50x) durchgeführt. Aus dieser Aufnahme ist die Anordnung der Fasern **3** auf der Oberfläche des Basismaterials **1** ersichtlich. Fig. 3 zeigt, dass sich durch die Verteilung der Fasern **3** eine regelmäßige Verteilung von Zwischenräumen zwischen den Fasern **3** ergibt, die mit einem mittleren Durchmesser von 40 µm bis 130 µm die optimale Größe für die Besiedlung mit Zellen haben.

Für die Rasterelektronenmikroskopische Aufnahme (**Fig. 4**) wurde eine Probe aus einem, wie zu Fig. 1 beschrieben hergestellten, Trägermaterial mit einer Schere ausgeschnitten, auf einer Aluminium-Halterung befestigt und im Vakuum mit Kohlenstoff bedampft. Die Aufnahme wurde bei einem Arbeitsabstand von 13 mm und einer Beschleunigungsspannung von 5 kV an einem Zeiss DSM 982 Gemini (Fa. Zeiss, Oberkochen, Deutschland) Rasterelektronenmikroskop gemacht. Fig. 4 zeigt, dass sich durch die elektrostatische Beflockung nahezu alle Fasern **3** in einer geordneten, nahezu senkrechten Ausrichtung zu der Oberfläche des Basismaterials **1** angeordnet sind. Die Fasern **3** ragen mit ihren unteren Enden in die Klebstoffschicht **2** hinein und sind dadurch fest mit dem Basismaterial **1** verbunden. Bedingt durch Kapillarkräfte an den Fasern **3** und den Trocknungsvorgang ist die Klebstoffschicht **2** direkt an den Fasern **3** etwas dicker. Die Klebstoffschicht **2** formt dadurch um das untere Ende der Faser **3** eine Art Sockel. Die Fasern **3** am vorderen Rand sind bedingt durch das Ausschneiden der Probe durch die Schere etwas zur Seite geknickt.

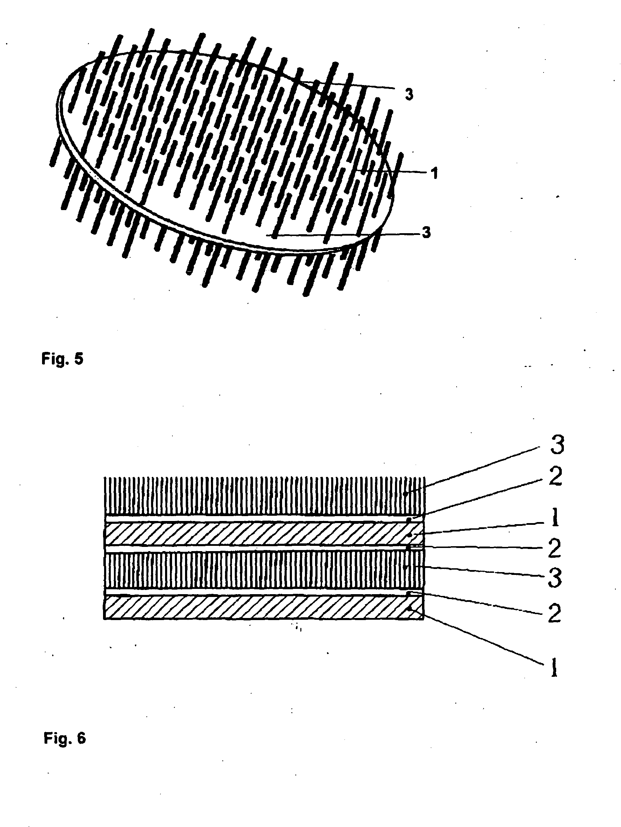

Das beidseitig mit Fasern **3** beflockte Trägermaterial aus **Fig. 5** ist aus den gleichen Materialien aufgebaut, wie die zu Fig. 1 beschrieben. Es enthält ebenfalls eine Klebstoffschicht zwischen den Fasern **3** und dem Basismaterial **1**, die aus Gründen der Übersicht nicht dargestellt ist. Zur Herstellung dieses Trägermaterials mit beidseitig beflocktem Basismaterial **1** wird ein Kollagen-Tape **1**, wie zu Fig. 1. beschrieben, zuerst auf einer Seite mit Fasern **3** elektrostatisch beflockt. Das so gebildete Trägermaterial wird anschließend umgedreht und auf der zweiten Seite, wie zu Fig. 1. beschrieben, ebenfalls mit Fasern 3 beflockt. Das beidseitig beflockte Tape **1** wird anschließend, wie zu Fig. 1 beschrieben, gefriergetrocknet und quervernetzt.

**Fig. 6** zeigt ein zweilagiges Trägermaterial. Dieses besteht aus zwei Trägermaterialien, in welchen die Basismaterialien **1** einseitig mit Fasern **3** beflockt sind und welche analog zu den in Fig. 1 beschriebenen aufgebaut sind. Des weiteren enthält das zweilagige Trägermaterial eine Klebstoffschicht **2** zwischen den beiden Trägermaterialien. Das Fasermaterial **3** besteht hier aus Polylactid (PLA), einem biokompatiblen und resorbierbaren Material, mit einem E-Modul von 5 bis 7 GPa. Die Fasern 3 haben eine Länge von 1 mm und einen Durchmesser von 30 µm.

Zur Herstellung des zweilagigen Trägermaterials wird wie folgt verfahren: Es werden zwei Trägermaterialien, analog zu Fig.1 beschrieben, hergestellt. Als Fasern **3** werden jedoch oben genannte Fasern **3** aus PLA verwendet. Auf die nicht-beflockte Seite des ersten Trägermaterials wird eine Gelatinelösung, die wie zu Fig. 1 beschrieben hergestellt ist, mit einer Klebstoffdicke **2** von 0,02 mm aufgebracht. Die mit Klebstoff **2** beschichtete Seite des ersten Trägermaterials wird nun auf die beflockte Seite des zweiten Trägermaterials aufgesetzt. Das so hergestellte zweilagige Trägermaterial wird, wie zu Fig. 1 beschrieben, gefriergetrocknet und quervernetzt.

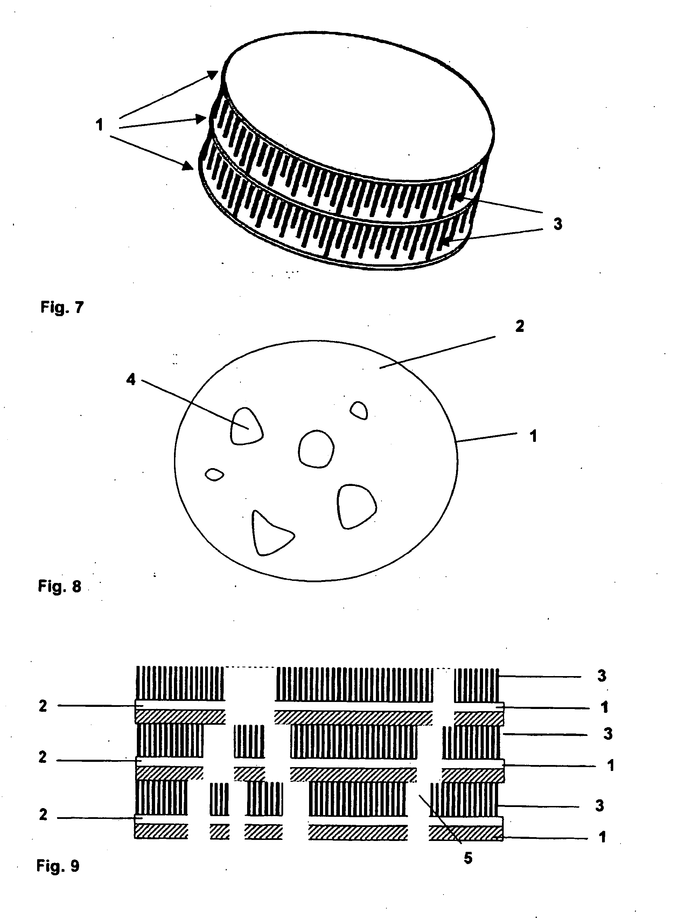

Das dreilage Trägermaterial aus **Fig. 7** ist aus zwei Trägermaterialien mit einseitig beflockten Basismaterialien **1** und einem Trägermaterial mit beidseitig beflocktem Basismaterial **1** zusammengesetzt. Die Trägermaterialien mit einseitig beflockten Basismaterialien **1** sind wie zu Fig. 1 beschrieben aufgebaut und hergestellt.Das Trägermaterial mit beidseitig beflocktem Basismaterial **1** ist wie zu Fig. 6 beschrieben aufgebaut und hergestellt. Zur Herstellung dieses mehrlagigen Trägermaterials werden die einzelnen Trägermaterialien mit einseitig beflockten Basismaterialien **1** jeweils mit ihrer Faserschicht **3** in eine Faserschicht **3** des Trägermaterials mit beidseitig beflocktem Basismaterial **1** gesteckt. Durch dieses Ineinanderstecken der Faserschichten **3** halten die Trägermaterialien analog des Klettverschlussprinzips zusammen, ohne dass es einer zusätzlichen Klebstoffschicht **2** bedarf.

Das in **Fig. 8** dargestellte partiell nicht beflockte Trägermaterial ist in gezielt ausgewählten Bereichen **4** nicht mit Fasern beflockt. Bezüglich der Materialien ist es, wie zu Fig. 1 beschrieben, aufgebaut und wie zu Fig. 1 beschrieben beflockt. Die nicht beflockten Bereiche **4** werden dadurch gebildet, dass mittels in der Flocktechnik üblichen Siebdruckschablonen entsprechend dem Muster der Siebdruckschablonen, ein wie zu Fig. 1 beschriebener Klebstoff **2** aufgetragen wird. Die durch das Muster partiell mit Klebstoff **2** beschichteten Basismaterialien **1** werden, wie zu Fig.1 beschrieben beflockt, wobei bei klebstofffreien Flächen keine Fasern **3** gebunden werden.

**Fig. 9** zeigt ein dreilagiges Trägermaterial mit Hohlräumen **5**. Dieses ist aus drei Trägermaterialien mit einseitig beflockten Basismaterialien **1** aufgebaut. Diese Trägermaterialien enthalten definierte, aber unterschiedliche Löcher (Durchbrüche). Das nachträgliche Ausschneiden hat den Vorteil, dass die Flockfasern am Lochrand nahezu senkrecht angeordnet sind.

Zur Herstellung dieses mehrlagigen Trägermaterials mit Hohlräumen **5** werden in drei Trägermaterialien, die, wie zu Fig.1 beschrieben aufgebaut und hergestellt sind, nach der Beflockung Löcher ausgestanzt. Die so perforierten Trägermaterialien werden übereinander gestapelt, wobei die Löcher z. T. übereinander angeordnet werden. Durch diese Anordnung werden in dem dreidimensionalen Mehrschichtträgermaterial röhrenförmige Hohlräume gebildet. Bei einer späteren Besiedlung mit Zellen dienen diese Hohlräume unter anderem der Nährstoff- und Sauerstoffzufuhr zu den Zellen.

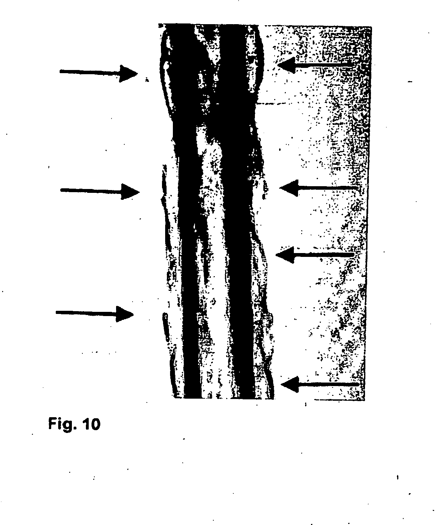

Die Lichtmikroskopische Aufnahme aus **Fig. 10** zeigt eine mit Fibroblasten besiedelten PHB-Faser in einem Trägermaterial. Die Anordnung einzelner Zellen wird durch Pfeile hervorgehoben.

Der Aufbau und die Herstellung eines Implantats mit dem erfindungsgemäßen Trägermaterial wird nachfolgend anhand des Beispiels eines Knorpelersatzmaterials näher erläutert.

Das Trägermaterial besteht hier aus einer Basismaterialschicht aus einem mineralisierten Hydroxylapatit-Kollagen-Tape mit einem Durchmesser von 7 cm und einer mittleren Dicke von 0,3 mm. Als Fasermaterial für die elektrostatische Beflockung werden biokompatible und resorbierbare Kurzfasern aus Polyhydroxybutyrat mit einer Faserlänge von 1 mm eingesetzt. Als Klebstoff wurde die Pharma-Gelatine der DGF Stoess AG verwendet. Das Trägermaterial wurde wie zu Fig. 1 beschrieben einseitig beflockt, gefriergetrocknet und eine Probe mit einem Durchmesser von 13 mm ausgestanzt. Das so hergestellte Trägermaterial zeichnet sich durch eine Flockfaserdichte von 260 Fasern/mm², einer Druckstabilität von σ_{D} = 0,11 MPa (linear elastisches Verhalten), einem Druckmodul von E = 0.21 MPa, einer Faserauszugsfestigkeit von σ_{F} = 0,46 MPa und Faserabstände zwischen 50 µm und 100 µm aus.

Auf die ausgestanzte Probe ist als Hüllstruktur ein strukturelastischer Gestrickschlauch aus Polyhydroxybutyrat aufgezogen. Der Gestrickschlauch wird aus Polyhydroxybutyrat-Fasern auf der Flachstrickmaschine CMS 211 der Fa. Stoll mit einer Maschinenfeinheit von E 18 gefertigt. Das umhüllte Trägermaterial wird durch Bestrahlung mit Gamma-Strahlung sterilisiert.

Zur Besiedelung mit Knorpelzellen wurde das umhüllte Trägermaterial zunächst für 24 Stunden in 5 ml sterilem Zellkulturmedium (DMEM, Sigma GmbH, München, Deutschland) eingelegt. Das Medium wird alle 6 Stunden ausgetauscht. Anschließend wird die Probe aus dem Medium herausgenommen und durch Auflegen auf ein steriles, saugfähiges Tuch etwas abgetrocknet. Die Probe wird nun mit der beflockten Seite nach oben in eine übliche 24er-Well-Zellkulturplatte überführt und eine Suspension von 1 × 10⁵ primären, humanen

Chondrozyten in 1 ml Zellkulturmedium wird mittels einer Pipette aufgebracht. Die Zellkulturplatte wird nun für **24** h in einem üblichen Zellkultur-Brutschrank bei Standardbedingungen inkubiert. Während dieser Zeit erfolgt die Adhäsion der Chondrozyten an das Trägermaterial. Anschließend wird das so besiedelte Trägermaterial in frisches Medium überführt und bis zur weiteren Verwendung bei Mediumwechsel drei Mal wöchentlich unter Standardbedingungen bis zur Implantation weiter kultiviert.

Während der Inkubation siedeln sich die Chondrozyten vorwiegend auf und zwischen den Fasern an. Durch eine Applikation von Ultraschall oder Druck auf die Oberfläche des umhüllten Trägermaterials wird eine mechanische Belastung *in vitro* simuliert, die der natürlichen Belastung der Chondrozyten im Gelenkknorpel *in vivo* nahe kommt. Die Flockfasern simulieren dabei die natürliche Matrix des Knorpels und nehmen ein Teil der

Belastung auf.

Bei der Implantation in den menschlichen Körper wird das umhüllte Trägermaterial an Bereichen eines Gelenks platziert an denen der Knorpel degeneriert oder defekt ist. Die mit Fasern beflockte Seite des Trägermaterials wird dabei in Richtung der Gelenkfläche angeordnet. Die unbeflockte Seite liegt dabei auf dem degenerierten Knorpel bzw. auf dem Knochen auf bzw. ist der Knochenseite zugewandt.

Für Bereiche der Gelenkfläche, die starke Lasten tragen, wird als Basismaterial des Trägermaterial anstelle des Kollagentapes ein Tape auf Basis von Polyhydroxybutyrat zur Herstellung des Trägermaterials verwendet. Der Flockprozess und die Besiedlung mit Chondrozyten erfolgt entsprechend.

Bei der Implantation wird das zellbesiedelte Trägermaterial hier mit der unbeflockten, relativ glatten Seite des Basismaterial in Richtung Gelenkfläche angeordnet. Durch diese Anordnung wird die Reibung zwischen Gelenk und Implantat reduziert.

## Patentansprüche

1. Trägermaterial für die Gewebe- und Zellkultur und die Herstellung von Implantaten oder Implantatmaterialien, bestehend aus biokompatiblen Materialien, **dadurch gekennzeichnet, dass** das Trägermaterial aus mindestens einem Basismaterial (1) besteht, das auf mindestens einer Seite mit Fasern (3) elektrostatisch beflockt ist, wobei die Fasern durch die elektrostatische Beflockung auf der Oberfläche des Basismaterials nahezu senkrecht angeordnet sind.

2. Trägermaterial nach Anspruch 1, **dadurch gekennzeichnet, dass** das Basismaterial (1) auf mindestens einer Seite mit Klebstoff (2) beschichtet ist.

3. Trägermaterial nach Anspruch 1 oder 2, **dadurch gekennzeichnet, dass** das Basismaterial (1) und/oder der Klebstoff (2) und/oder die Fasern (3) aus resorbierbarem Material bestehen.

4. Trägermaterial nach Anspruch 3, **dadurch gekennzeichnet, dass** die resorbierbaren Fasern (3) aus resorbierbarem Polymer, vorzugsweise aus der Gruppe Polylactid, Polycaprolacton, Polyhydroxybutyrat und Polyglycolid, oder Derivate und/oder Copolymere dieser Polymere bestehen, und dass das resorbierbare Basismaterial (1) und/oder der resorbierbare Klebstoff (2) ausgewählt ist aus der Gruppe Kollagen, Kollagenderivate, Hyaluronsäure, Chitosan und Gelatine oder aus Kompositen von Materialien aus der vorgenannten Gruppe.

5. Trägermaterial nach einem der Ansprüche 1 bis 4, **dadurch gekennzeichnet, dass** die Länge der Fasern (3) zwischen 0,3 mm und 3 mm und/oder der Durchmesser der einzelnen Fasern (3), zwischen 10 µm und 200 µm beträgt.

6. Trägermaterial nach einem der Ansprüche 1 bis 5, **dadurch gekennzeichnet, dass** die Fasern (3) auf dem Basismaterial (1) in einem mittleren Abstand von 40 µm bis 250 µm angeordnet sind.

7. Trägermaterial nach einem der Ansprüche 1 bis 6, **dadurch gekennzeichnet, dass** zumindest ein Teil der Fasern (3) Hohlfasern sind.

8. Trägermaterial nach einem der Ansprüche 1 bis 7, **dadurch gekennzeichnet, dass** es mit Zellen besiedelt ist.

9. Mehrlagiges Trägermaterial, **dadurch gekennzeichnet, dass** wenigstens zwei biokompatible Trägermaterialien nach einem der Ansprüche 1 bis 8 miteinander verbunden oder übereinander gestapelt und/oder mit ihren Faserschichten (2) ineinander gesteckt sind.

10. Mehrlagiges Trägermaterial nach Anspruch 9, **dadurch gekennzeichnet, dass** es Hohlräume (5) oder ein Hohlraumsystem enthält.

11. Verwendung eines Trägermaterials nach einem der Ansprüche 1 bis 10 für die Gewebe- und/oder Zellkultur und/oder die Herstellung von Implantaten oder Implantatmaterialien.

12. Implantat oder Implantatmaterial, **dadurch gekennzeichnet, dass** es ein Trägermaterial oder ein mehrlagiges Trägermaterial nach einem der Ansprüche 1 bis 10 enthält.

13. Implantat oder Implantatmaterial nach Anspruch 12, **dadurch gekennzeichnet**, das Trägermaterial von einer Hülle, vorzugsweise einer Textilstruktur, einer Folie oder einem Tape, umgeben ist.

14. Verfahren zur Herstellung einer Gewebe- oder Zellkultur oder eines Implantatmaterials mit den folgenden Schritten:
a.) Herstellung eines Trägermaterials nach einem der Anspruche 1 bis 10 durch elektrostatisches Beflocken eines Basismaterials (1) auf mindestens einer Seite mit Fasern (3),
b.) Inkubation des Trägermaterials mit Zellen zur Zellbesiedelung des Trägermaterials und Ausbildung einer extrazellulären Matrix,
wobei nach der Ausbildung der extrazellulären Matrix das Basismaterials (1) gegebenenfalls wieder entfernt wird.

15. Implantatmaterial, welches Zellen und eine extrazelluläre Matrix enthält, hergestellt durch die Schritte:
a.) Herstellung eines Trägermaterials nach einem der Ansprüche 1 bis 10 durch elektrostatisches Beflocken eines Basismaterials (1) auf mindestens einer Seite mit Fasern (3),
b.) Inkubation des Trägermaterials mit Zellen zur Zellbesiedelung des Trägermaterials und Ausbildung einer extrazellulären Matrix,
c.) Entfernen des Basismaterials (1) nach der Ausbildung der extrazellulären Matrix.

16. Verwendung des Verfahrens der elektrostatischen Beflockung zur Herstellung eines biokompatiblen Trägermaterials nach einem der Ansprüche 1 bis 10 für die Gewebe- und Zellkultur und/oder die Herstellung von Implantatmaterialien.

## Claims

1. Scaffold for tissue culture and cell culture and the production of implants or implant materials, consisting of biocompatible materials, **characterised in that** the scaffold consists of at least one base material (1), which is electrostatically flocked with fibres (3) on at least one side, whereas hrough the electrostatic flocking the fibres are arranged almost vertically on the surface of the base material.

2. Scaffold according to claim 1, **characterised in that** the base material (1) is coated on at least one side with an adhesive (2).

3. Scaffold according to claim 1 or 2, **characterised in that** the base material (1) and/or the adhesive (2) and/or the fibres (3) consists of resorbable material.

4. Scaffold according to claim 1, **characterised in that** the resorbable fibres (3) consist of a resorbable polymer, preferably from the group polyactide, polycaproplactone, polyhydroxybutyate and polyglycolide, or derivatives and/or copolymers of these polymers, and **in that** the resorbable base material(1) and/or the resorbable adhesive (2) is selected from the group collagen, collagen derivatives, hyaluronic acid, chitosan and gelatine or from composites of materials from the above mentioned group.

5. Scaffold according to one of the claims 1 to 4, **characterised in that** the length of the fibres (3) lies between 0.3 mm and 3 mm and/or the diameter of the individual fibres (3) lies between 10 µm and 200 µm.

6. Scaffold according to one of the claims 1 to 5, **characterised in that** the fibres (3) are arranged on the base material (1) with a mean distance between 40 µm and 250 µm.

7. Scaffold according to one of the claims 1 to 6, **characterised in that** at least a part of the fibres (3) are hollow fibres.

8. Scaffold according to one of the claims 1 to 7, **characterised in that** it is colonised with cells.

9. Multi-layered scaffold, **characterised in that** at least two biocompatible scaffolds according to one of the claims 1 to 8 are connected or stacked on top of one another and/or with their fibre layers (2) stuck into one another.

10. Multi-layered scaffold according claim 9, **characterised in that** it contains hollow spaces (5) or a system of hollow spaces.

11. Use of a scaffold consisting of a base material (1), which is electrostatically flocked on at least one side with fibres (3), for tissue culture and/or cell culture and/or the production of implants or implant materials.

12. Implants or implant material **characterised in that** it contains a scaffold or multi-layered scaffold according to one of the claims 1 to 10.

13. Implants or implant material according to claim 12, **characterised in that** the scaffold is surrounded by a shell, preferably a textile structure, film or tape.

14. Process for the production of a tissue culture or cell culture or an implant material with the following steps:
a) Production of a scaffold according to one of the claims 1 to 10 through the electrostatic flocking of a base material (1) with fibres (3) on at least one side
b) Incubation of the scaffold with cells for the colonisation of the scaffold with cells and formation of an extra-cellular matrix,
whereby the base material (1) is removed where appropriate after the formation of the extra-cellular matrix.

15. Implant material, which contains cells and an extra-cellular matrix, produced by the following steps:
a) Production of a scaffold according to one of the claims 1 to 10 by the electrostatic flocking of a base material (1) with fibres (3) on at least one side,
b) Incubation of the scaffold with cells for the colonisation of the scaffold with cells and the formation of an extra-cellular matrix,
c) Removal of the base material (1) after the formation of the extra-cellular matrix.

16. Use of the process of electrostatic flocking fort he production of a scaffold according to one of the claims 1 to 10 for tissue culture and cell culture and/or the production of implants or implant materials.

## Revendications

1. Matériau de support pour la culture de tissus et de cellules et la fabrication d'implants ou de matériaux d'implant, composé de matériaux biocompatibles, **caractérisé en ce que** le matériau de support se compose d'au moins un matériau de base (1) qui est floqué par voie électrostatique sur au moins une face avec des fibres (3), les fibres étant disposées à la surface du matériau de base de manière approximativement perpendiculaire par le flocage électrostatique.

2. Matériau de support selon la revendication 1, **caractérisé en ce que** le matériau de base (1) est revêtu de produit adhésif (2) sur au moins une face.

3. Matériau de support selon la revendication 1 ou 2, **caractérisé en ce que** le matériau de base (1) et/ou le produit adhésif (2) et/ou les fibres (3) sont composés de matériau résorbable.

4. Matériau de support selon la revendication 3, **caractérisé en ce que** les fibres résorbables (3) sont composées de polymère résorbable, de préférence du groupe polylactide, polycaprolactone, polyhydroxybutyrate et polyglycolide ou de dérivés et/ou de copolymères de ces polymères, et que le matériau de base résorbable (1) et/ou le produit adhésif résorbable (2) est sélectionné parmi le groupe collagène, dérivés de collagène, acide hyaluronique, chitosane et gélatine ou parmi des composites de matériaux du groupe précité.

5. Matériau de support selon l'une des revendications 1 à 4, **caractérisé en ce que** la longueur des fibres (3) est comprise entre 0,3 mm et 3 mm et/ou le diamètre des différentes fibres (3) est compris entre 10 µm et 200 µm.

6. Matériau de support selon l'une des revendications 1 à 5, **caractérisé en ce que** les fibres (3) sont disposées sur le matériau de base (1) avec un écartement moyen de 40 µm à 250 µm.

7. Matériau de support selon l'une des revendications 1 à 6, **caractérisé en ce qu'**au moins une partie des fibres (3) est composée de fibres creuses.

8. Matériau de support selon l'une des revendications 1 à 7, **caractérisé en ce qu'**il est colonisé par des cellules.

9. Matériau de support multicouche, **caractérisé en ce qu'**au moins deux matériaux de support biocompatibles selon l'une des revendications 1 à 8 sont reliés ensemble ou empilés l'un sur l'autre et/ou emboîtés l'un dans l'autre par leurs couches de fibres (2).

10. Matériau de support multicouche selon la revendication 9, **caractérisé en ce qu'**il contient des cavités (5) ou un système de cavités.

11. Utilisation d'un matériau de support selon l'une des revendications 1 à 10 pour la culture de tissus et de cellules et/ou la fabrication d'implants ou de matériaux d'implant.

12. Implant ou matériau d'implant, **caractérisé en ce qu'**il contient un matériau de support ou un matériau de support multicouche selon l'une des revendications 1 à 10.

13. Implant ou matériau d'implant selon la revendication 12, **caractérisé en ce que** le matériau de support est entouré par une enveloppe, de préférence une structure textile, une feuille ou une bande.

14. Procédé de fabrication d'une culture de tissus ou de cellules ou d'un matériau d'implant comprenant les étapes suivantes :
a.) fabrication d'un matériau de support selon l'une des revendications 1 à 10 par flocage électrostatique d'un matériau de base (1) sur au moins une face avec des fibres (3),
b.) incubation du matériau de support avec des cellules pour la colonisation cellulaire du matériau de support et la formation d'une matrice extracellulaire,
le matériau de base (1) étant, le cas échéant, de nouveau éliminé après la formation de la matrice extracellulaire.

15. Matériau d'implant, lequel contient des cellules et une matrice extracellulaire, obtenu par les étapes suivantes :
a.) fabrication d'un matériau de support selon l'une des revendications 1 à 10 par flocage électrostatique d'un matériau de base (1) sur au moins une face avec des fibres (3),
b.) incubation du matériau de support avec des cellules pour la colonisation cellulaire du matériau de support et la formation d'une matrice extracellulaire,
c.) élimination du matériau de base (1) après la formation de la matrice extracellulaire.

16. Utilisation du procédé de flocage électrostatique pour fabriquer un matériau de support biocompatible selon l'une des revendications 1 à 10 pour la culture de tissus et de cellules et/ou la fabrication de matériaux d'implant.
